# EUROPÄISCHE PATENTSCHRIFT

(11) **EP 1 480 602 B1**
(45) Veröffentlichungstag und Bekanntmachung des Hinweises auf die Patenterteilung: **14.01.2009**
(21) Anmeldenummer: 03745258.8
(22) Anmeldetag: 25.02.2003
(51) Int. Cl.: A61K 8/06, A61Q 19/00

(54) **VERFAHREN ZUR HERSTELLUNG VON EMULSIONEN**
METHOD FOR PRODUCING EMULSIONS
PROCEDE POUR LA PRODUCTION D'EMULSIONS

(30) Priorität: 26.02.2002 DE 10208265
(43) Veröffentlichungstag der Anmeldung: 01.12.2004
(73) Patentinhaber: Beiersdorf AG, 20245 Hamburg (DE)
(72) Erfinder: BINDER, Wolfgang, 22457 Hamburg (DE); MONTERO, Angel, E-28050 Madrid (ES); RODRIGUEZ NUNEZ, Ramon, E-28050 Madrid (ES)
(86) Internationale Anmeldenummer: PCT/EP2003/001885
(87) Internationale Veröffentlichungsnummer: WO 2003/082220

(56) Entgegenhaltungen:
- WO-A-98/32413
- WO-A-99/49738
- DE-A- 19 923 785
- GB-A- 1 537 112

## Beschreibung

Die vorliegende Erfindung betrifft ein Verfahren zur Herstellung von kosmetischen und/oder dermatologischen Zubereitungen, insbesondere von Emulsionen, PIT-Emulsionen und waschaktive Substanzen enthaltende Zubereitungen.

Kosmetische und/oder dermatologische Zubereitungen in Form von Emulsionen, PIT-Emulsionen und waschaktive Substanzen enthaltenden Zubereitungen sind weit verbreitet. Emulsionen können W/O oder O/W oder auch multiple Emulsionen, das heißt mehr als zwei Phasen enthaltende Emulsionen sein. Sie treten am Markt in Form von Cremes, Lotionen, aber auch als schweißhemmende, Körpergeruch vermindernde, Reinigungs- und Sonnenschutzzubereitungen auf. PIT-Emulsionen sind besondere Formen von Emulsionen. Sie sind durch den Weg ihrer Herstellung und die daraus resultierenden Tröpfchengrößen gekennzeichnet. Zur Herstellung werden Emulgatoren bzw. Emulgator Systeme eingesetzt, die ihre Polarität abhängig von der Temperatur so ändern, daß während der Herstellung eine Phaseninversion auftritt. Durch diese Phaseninversion werden besondere Produkteigenschaften erzielt, wie beispielsweise ein besonderes optisches Erscheinungsbild oder eine außergewöhnlich geringe Viskosität. Derartige Zubereitungen eignen sich beispielsweise als sprühbare Hautpflege- oder Sonnenschutzemulsionen. Waschaktive Substanzen enthaltende Zubereitungen schließlich werden als Körper- oder Haarreinigungsmittel sowie als Geschirrspulmittel eingesetzt.

Üblicherweise werden derartige Zubereitungen in einem absatzweisen Verfahren hergestellt, meist in einem Mischer, in den die Ausgangsstoffe eingebracht werden und das Zwischen- oder Endprodukt nach einer bestimmten Betriebszeit entnommen werden. Dabei erfolgen in diesem einen Apparat nacheinander alle Verfahrensschritte, die zur Herstellung des Produktes erforderlich sind: Dosieren, Mischen, Heizen/Kühlen, Emulgieren, Kühlen. Oft wird bei der Entnahme das Produkt noch einer anschließenden Homogenisierung unterworfen. Obwohl in der Lebensmitteltechnik kontinuierliche Anlagen zur Herstellung von Emulsionen wie Joghurt oder Mayonnaise weit verbreitet sind, werden kosmetische und/oder dermatologische Zubereitung nur in Ausnahmefällen kontinuierlich hergestellt. Dies ist darin begründet, daß die Anforderungen an die Stabilität kosmetischer Produkte weit höher sind und diese aufgrund ihrer komplexeren Zusammensetzung aus zahlreichen unterschiedlichen Komponenten weit schwieriger stabil herstellbar sind. So wird beispielsweise von einem Joghurt eine Stabilität im Bereich von wenigen Wochen erwartet, während kosmetische Emulsionen über mindestens 30 Monate stabil sein sollen.

Anlagen, die in absatzweisen Verfahren betrieben werden, haben neben der vorteilhaften Flexibilität hinsichtlich der herstellbaren Produkte eine Reihe von Nachteilen. Die erforderlichen langen Ansatzzeiten führen zu erhöhten Produktionskosten. Es besteht eine Verkeimungsgefahr, da die Anlagen oft entleert und befüllt werden müssen. Die Verkeimungsgefahr kann begrenzt werden, indem die Produkttemperaturen niedrig gehalten werden. Dies wird erreicht, indem kalte wässrige Phasen eingesetzt werden. Alternativ kann auch ein Wärmetauscher nachgeschaltet werden. Zumeist werden größere Luftmengen in das System eingetragen, was unerwünscht ist.

Bekannte kontinuierliche Verfahren zeichnen sich dadurch aus, daß die einzelnen Phasen gleichzeitig in ein Hochleistungs-Emulgiergerät dosiert werden. Dort erfolgt unter hohem Energieeintrag der Emulgier- und Homogenisiervorgang, wobei hohe Scherkräfte auftreten. Das Auftreten hoher Scherkräfte vermag jedoch in den Zubereitungen enthaltene Polymere zu schädigen. Durch die gleichzeitige Dosierung aller Komponenten sind diese über längere Zeiträume höheren Temperaturen ausgesetzt. Dadurch ist der Einsatz temperaturempfindlicher Substanzen nur eingeschränkt möglich. Solche Substanzen sind beispielsweise kosmetische Wirk- und Funktionsstoffe wie Duftstoffe, Vitamine, Co-Enzyme, Peptide, Enzyme, Nukleinsäuren, Pflanzenextrakte, Konservierungsstoffe wie zum Beispiel die aus 1,2-Dibrom-2,4-dicyanobutan und 2-Phenoxyethanol.

Kontinuierliche Anlagen, mit denen sich eine Vielzahl verschiedener kosmetischer und/oder dermatologischer Zubereitungen herstellen lassen, sind zwar wünschenswert, aber bisher nicht bekannt. Insbesondere eine Anlage, auf der sich sowohl niedrigviskose Emulsionen, Lotionen, Cremes und Körper- und Haarreinigungszubereitungen sowie Geschirrspülmittel herstellen ließen, stellte eine deutliche Verbesserung des Standes der Technik dar.

Bisher gelang es nicht, PIT-Emulsionen in kontinuierlichen Verfahren herzustellen, da die Tröpfchen trotz hohen Energieeintrages zu groß blieben. Kontinuierlich hergestellte Cremes und flüssige Emulsionen waren zumeist nicht ausreichend stabil. Bei der Herstellung von waschaktive Substanzen enthaltenden Zubereitungen führt die Anwendung kontinuierlicher Verfahren meist dazu, daß durch ungenügende Durchmischung Inhomogenitäten entstehen Daher sind transparente Zubereitungen nur schwierig erhältlich, da derartige Inhomogenitäten oft zu Eintrübungen führen. Bei der Herstellung von O/W-Emulsionen haben sich bisher kontinuierliche Verfahren nicht durchsetzen können, da es nicht gelang, Produkte hoher Qualität zu erreichen: meist waren die Emulsionen nicht stabil oder neigten zu Ausölungen. Als Ursache für dieses Verhalten ist anzunehmen, daß durch den Einsatz von statischen Mischern allein keine homogene Tröpfchengrößenverteilungen zu erreichen sind.

Der Artikel "Eine Anlage zum kontinuierlichen Emulgieren" in der Zeitschrift Verfahrenstechnik, Heft 1-2 aus dem Jahr 1986 beschreibt beispielsweise ein kontinuierliches Herstellverfahren zur Herstellung von W/O- und O/W-Cremes. Die diskontinuierlich angesetzten Vorprodukte durchlaufen ein Dosiersystem, einen dynamischen Mischer und einen statischen Mischer. Dabei wird ein hot/cold-Verfahren realisiert, in dem die Vorprodukte Wasser- und Fettphase kalt bzw. heiß in den Prozess eintreten. Im dynamischen Mischer wird die Emulsion erzeugt, im statischen Mischer erfolgt eine Homogenisierung, wodurch die Tropfengrößenverteilung eingestellt wird. Diese Anlage eignet sich zur Herstellung von Hautcremes, Körperlotionen, Mayonnaise und Saucen.

Dabei erfolgt der Homogenisiervorgang bei 40 bis 75°C, wünschenswert wäre aber, diesen Schritt bei niedrigen Temperaturen durchzuführen, da temperaturempfindliche Bestandteile der Formulierungen wie Geruchs- und Aromastoffe oder Wirkstoffe wie Vitamine möglichst keinem thermischen Stress ausgesetzt werden sollen.

Ausgehend hiervon war Aufgabe der vorliegenden Erfindung, ein Verfahren zu finden, welches den Nachteilen des Standes der Technik abhilft.

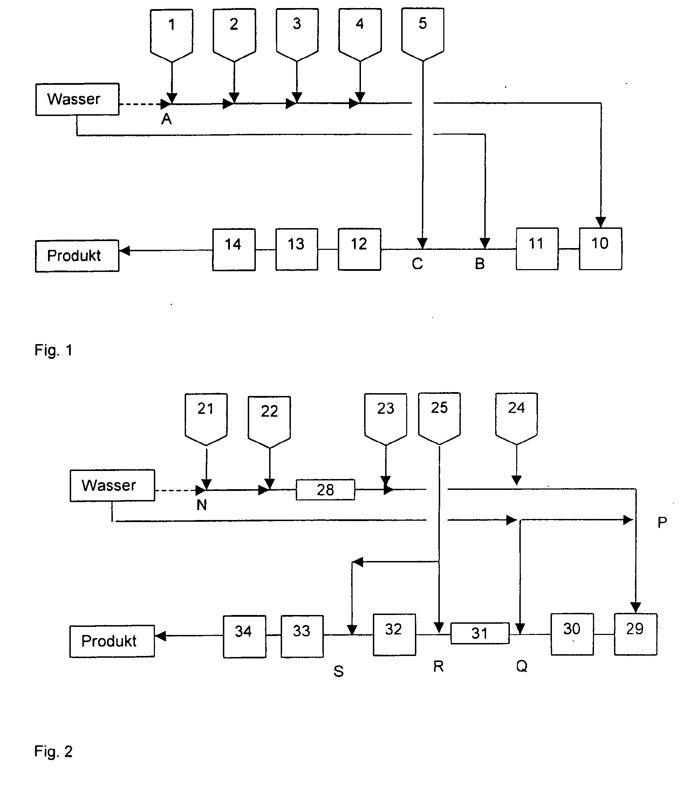

Es hat sich für den Fachmann nicht vorhersehbar herausgestellt, daß ein kontinuierliches Herstellverfahren, wie es in Fig. 1 dargestellt ist, für kosmetische und/oder dermatologische Zubereitungen, die temperaturempfindliche Inhaltstoffe enthalten, gekennzeichnet durch eine Abfolge folgender Verfahrensschritte
(a) Emulgieren in Mischapparaten (11),
(b) Einstellen einer Temperatur der Mischung von weniger 40°C durch Zugabe (B) von wässriger Phase mit einer gegenüber der Mischung niedrigeren Temperatur,
(c) Zugabe (C) von Parfümöl und/oder temperaturempfindlichen Wirkstoffen,
(d) Homogenisieren in Apparaten (13) im Temperaturbereich von 20 bis 50°C, besonders bevorzugt bei 28 bis 40°C, den Nachteilen des Standes der Technik abhilft. Ebenso hilft auch ein Herstellverfahren für kosmetische und/oder dermatologische Zubereitungen, die temperaturempfindliche Inhaltstoffe enthalten, wie in Fig. 2 dargestellt und gekennzeichnet dadurch, daß
   (1) es kontinuierlich durchgeführt wird sowie
   (2) durch eine Abfolge folgender Verfahrensschritte

(a) Emulgieren in Mischapparaten (30), in Kombination mit statischen Mischern (28, 11) bzw. Homogenisatoren (29, 33),
(b) Einstellen einer Temperatur der Mischung von 55 - 35°C durch Zugabe (P,Q) von wässriger Phase von 15 - 50°C mit einer gegenüber der Mischung niedrigeren Temperatur,
(c) Zugabe (R,S) von Parfümöl und/oder temperaturempfindlichen Wirkstoffen bei unterschiedlichen Temperaturen,
(d) Homogenisieren in Apparaten (29, 33) im Temperaturbereich von entweder 50 bis 80°C, besonders bevorzugt bei 60 bis 7.0°C oder 20 bis 50°C, besonders bevorzugt 28 bis 45°C ganz besonders bevorzugt 30 bis 40°C,
(e) Schrittweises (stufenweises) Kühlen währendes des Prozesses (32,34), den Nachteilen des Standes der Technik ab. Dabei ist es besonders bevorzugt, wenn die eintretenden Vorprodukte zuvor auf Temperaturen von 40 bis 100°C, besonders bevorzugt bei 50 bis 90°C temperiert wurden

Dabei ist es bevorzugt, wenn als weiterer nachgeschalteter Verfahrensschritt (14), wie in Fig. 1 dargestellt oder (32, 34), wie in Fig. 2 dargestellt, das Verfahrensprodukt auf höchstens 28 bis 30°C gekühlt wird. Bevorzugt ist es auch, wenn die Vorprodukte bei Temperaturen von 40 bis 100°C, besonders bevorzugt bei 50 bis 90°C vermischt werden, bevor sie in den ersten Misch- oder Homogenisierapparat eintreten. Weiterhin bevorzugt ist es, wenn beim Durchlaufen des Homogenisierapparates (13), wie in Fig. 1 dargestellt, die Temperatur der austretenden Mischung 2 bis 60°C oder beim Durchlaufen des Homogenisierapparates (29, 33), wie in Fig. 2 dargestellt, die Temperatur der austretenden Mischung 2 bis 10°C, bezogen auf die Temperatur der eintretenden Mischung ansteigt.

Besonders bevorzugt ist es, wenn der Emulgiervorgang in zwei unterschiedlichen Mischapparaten (10) und (11), wie in Fig. 1 dargestellt, durchgeführt wird. Ganz besonders bevorzugt ist es, wenn der Emulgiervorgang in einem statischen Mischer (10) und einem Schlaufenmischer (11) (Fig. 1) oder (29) und (33) (Fig. 2)durchgeführt wird.

Besonders bevorzugt ist es, wenn der Homogenisiervorgang in einem Schlaufenmischer (30) und einem Homogenisator (33) durchgeführt wird, wie in Fig. 2 dargestellt.

Besonders bevorzugt ist es, wenn der Homogenisiervorgang in zwei unterschiedlichen Apparaten (12) und (13) durchgeführt wird, wie in Fig. 1 dargestellt. Ganz besonders bevorzugt ist es, wenn der Homogenisiervorgang in einem statischen Mischer (12) und einem Homogenisator (13) durchgeführt wird.

Ganz besonders bevorzugt ist es, wenn der Emulgiervorgang in einem Schlaufenmischer (30) in Kombination mit einem oder mehreren Homogenisatoren (29, 33), sowie statischen Mischern (28, 31) durchgeführt wird, wie in Fig. 2 dargestellt.

Die Erfindung umfasst auch Emulsionen, PIT-Emulsionen und waschaktive Substanzen enthaltende Produkte erhältlich durch ein Verfahren nach mindestens einer der beschriebenen Varianten. Bevorzugt enthalten derartige Emulsionen, PIT-Emulsionen und waschaktive Substanzen enthaltende Produkte bzw. kommen in derartigen Verfahren zum Einsatz als temperaturempfindliche Inhaltsstoffe wie Duftstoffe, Vitamine, Co-Enzyme, Peptide, Enzyme, Nukleinsäuren, Pflanzenextrakte, Konservierungsstoffe.

Durch das erfindungsgemäße Verfahren können besonders hohe Durchsatzleistungen der verwendeten Anlagen erreicht werden: bisher lagen die Kapazitätsgrenzen üblicher Anlagen bei 3 t/h, während mit der erfindungsgemäßen Anlage bis zu 10 t/h erreicht werden können. Dabei ist das Verfahren sehr universell für völlig verschiedenartige Produktgruppen geeignet: neben W/O- und O/W-Emulsionen lassen sich auch PIT-Emulsionen und waschaktive Substanzen enthaltende Produkte besonders kostengünstig auf ein und derselben Anlage herstellen, wobei die Produkte besonders stabil und auch über lange Zeiträume lagerfähig sind. Aufgrund der universellen Einsetzbarkeit der Anlage kann zu besonders niedrigen Kosten produziert werden. Bei PIT-Emulsionen können besonders geringe Tropfengrößen erreicht werden, die sonst nur in Laborversuchen langzeitlagerstabil hergestellt werden können.

Dies ist insbesondere ein großer Vorteil, wenn Sonnenschutzformulierungen auf Basis von PIT-Emulsionen hergestellt werden sollen: auf diese Weise lassen sich besonders viel Lichtschutzmittel einarbeiten und so besonders hohe Lichtschutzfaktoren von bis zu 40 und mehr erreichen.

Es ist von Vorteil, in dem erfindungsgemäßen Verfahren als Schlaufenmischer einen Apparat zu verwenden, der sich durch einen vom Produktauslauf entfernt angeordneten Produktzulauf, eine Fördereinrichtung wie eine mehrgängige Förderschnecke, die sich in einem innenliegenden Leitrohr befindet kennzeichnet, wobei die Durchmischung des Produkts dadurch bewirkt wird, daß das durch die innenliegende Fördereinrichtung geförderte Volumen ein Vielfaches des durch den Zulauf zugeführten Volumens beträgt und so ein Zwangsumlauf außerhalb des Leitrohres entgegen der Förderrichtung innerhalb des Leitrohres entsteht. Besonders bevorzugt ist es, einen Mischer vom Typ Burdosa DMT 320 zu verwenden. Derartige Mischer fanden bisher Verwendung zur Herstellung von Orangensaftkonzentrat, Joghurt, Salatsaucen der anderen Lebensmitteln und erlauben eine sehr variable Anpassung der Prozessparameter an die Erfordernisse. So kann neben reinem Mischerbetrieb auch emulgiert oder aufgeschäumt werden.

Es ist von Vorteil, in dem erfindungsgemäßen Verfahren als weiteren Mischer einen Apparat zu verwenden, der gleichzeitig als Homogenisator wirkt. Bevorzugt findet ein Homogenisator vom Typ Becomix DH 500, Firma Berents, Stuhr, DE, Verwendung. Vorteilhaft ist es insbesondere, einen Hochdruckhomogenisator bestehend aus einer Hochdruckpumpe, einer strukturierten Packung und einem Ventil, wie es beispielsweise in der europäischen Patentanmeldung 810025 beschrieben ist, zu verwenden.

Weiter vorteilhaft ist es, statt des Mischers (10) eine Kombination zweier Mischapparate einzusetzen, insbesondere einen statischen Mischer und einen dynamischen Mischer. In diesem Fall steigt die Temperatur der austretenden Mischung beim Durchlaufen der Kombination aus Mischapparaten um 2 bis 60°C an, bezogen auf die Temperatur der eintretenden Mischung.

Durch Hochdruckhomogenisierung wird insbesondere vorteilhaft ein Aufheizen in einem separaten Verfahrensschritt überflüssig, da durch den Energieeintrag der Homogenisierung das homogenisierte Gut sehr effektiv gleichzeitig aufgeheizt wird.

Ein weiterer Vorteil des erfindungsgemäßen Prozesses ist die Eigenschaft, daß die Reinigung der Anlage bei einem Wechsel des Produkts sehr einfach ist. Lässt man eine Reinigungslösung zulaufen und fährt diese auf geeignete Weise im Kreislauf, so entfällt eine Demontage oder sonst wie aufwändige Reinigung. Solche Apparate bezeichnet man auch als cip-fähig (engl.: clean in process).

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

Die Beispiele (1) bis (5) beziehen sich auf Fig. 1, die Beispiele (6) bis (10) auf Fig. 2. In Fig 2 bedeuten 28 = Statischer Mischer, 29 = Beco Homogenisator, 30 = Schlaufenmischer, 31 = Statischer Mischer, 32 = Wärmetauscher, 33 = Beco Homogenisator, 34 = Wärmetauscher

### (1) Herstellung einer wirkstoffhaltigen Creme

| | | |
|---|---|---|
| Behälter 2 | 44,168 | Vollentsalztes Wasser |
| | 7,500 | Glycerin |
| | 0,200 | Natriumhydroxidlösung 45% |
| | | |
| Behälter 1 | 3,000 | Glycerylstearatcitrat |
| | 2,000 | Caprylic/Capric Triglycerid |
| | 2,000 | Tridecyl Stearat |
| | 1,100 | Stearylalkohol |
| | 1,100 | Cetylalkohol |
| | 1,500 | Hydryierte Kokosfettglyceride |
| | 0,010 | Ceramid 3 |
| | | |
| Kaltwasser | 20,270 | Vollentsalztes Wasser |
| | | |
| Behälter 4 | 3,000 | Dicaprylylether |
| | 0,400 | Carbomer |
| | | |
| Behälter 3 | 0,002 | Ubichinon |
| | 10,000 | Cyclomethicon |
| | | |
| Behälter 5 | 3,000 | Ethanol |
| | 0,500 | Konservierungsstoffe |
| | 0,250 | Parfum |
| | | |

Zunächst werden folgende Phasen in Ansatzbehältern vorgelegt: in Ansatzbehälter (1) eine auf 60 bis 95°C temperierte Ölphase, in Ansatzbehälter (2) eine auf 80°C temperierte Wasserphase, in Ansatzbehälter (3) eine elektrolythaltige Phase, in Ansatzbehälter (4) eine Carbomerphase und in Ansatzbehälter (5) eine Parfümöl und Wirkstoffe enthaltende Phase. Aus den Ansatzbehältern wird kontinuierlich dosiert. Die Ölphase aus Ansatzbehälter (1) wird zunächst mit der Wasserphase aus Ansatzbehälter (2) vereinigt, anschließend werden die wirkstoffhaltige Phase aus Ansatzbehälter (3) und die Carbomerphase aus Ansatzbehälter (4) zugegeben. Die Mischung durchläuft einen statischen Rohrmischer (10) Typ MS2G, Bran + Luebbe und wird anschließend in einem Schlaufenmischer (11) Typ Burdosa DMT 320 bei 500 Umdrehungen pro Minute emulgiert. Die austretende Emulsion hat eine Temperatur von 53,1°C, wird an Punkt (B) mit Kaltwasser schlagartig auf 35-38°C gekühlt und die Parfümöl und Wirkstoffe enthaltende Phase aus dem Ansatzbehälter (5) an Punkt (C) zugegeben. Nach dem Durchlaufen eines weiteren statischen Mischers (12), Typ MS2G, Bran + Luebbe, wird die Emulsion in einem Homogenisator (13) Typ Becomix DH 500, Firma Berents, homogenisiert, wobei die Temperatur um 2 bis 10°C ansteigt. Anschließend wird durch Wärmetauscher (14) auf 30°C abgekühlt und das Produkt abgefüllt. Es wird ein Durchsatz von 2 t/h erreicht.

### (2) Herstellung einer Softcreme

| | | |
|---|---|---|
| Behälter 1 | 1,500 | Paraffinöl |
| | 2,500 | Stearinsäure |
| | 2,000 | Petrolatum |
| | 3,500 | Myristylalkohol |
| | 1,500 | Myristylmyristat |
| | 1,200 | Glycerylstearat |
| | 1,000 | Hydryierte Kokosfettglyceride |
| | 0,100 | Cetylphosphat |
| | 0,350 | Konservierungsstoffe |
| | | |
| Behälter 2 | 23,630 | Entsalztes Wasser |
| | 3,500 | Glycerin |
| | | |
| Behälter 3 | 4,800 | Entsalztes Wasser |
| | 0,600 | Natriumhydroxidlösung 45% |
| | | |
| Behälter 4 | 0,750 | Dimethicon |
| | 0,300 | Carbomer |
| | 18,750 | Entsalztes Wasser |
| | 2,800 | Ethanol, denaturiert |
| | | |
| Behälter 5 | 0,500 | Tocopherylacetat |
| | 0,350 | Polyglyceryl-2-caprat |
| | 0,200 | Ethanol |
| | 0,170 | Parfum |
| | | |
| Kaltwassser | 30,000 | Entsalztes Wasser |

Zunächst werden folgende Phasen in Ansatzbehältern vorgelegt: in Ansatzbehälter (1) eine auf 60 bis 95°C temperierte Ölphase, in Ansatzbehälter (2) eine auf 80°C temperierte Wasserphase, in Ansatzbehälter (3) eine NaOH-haltige Phase, in Ansatzbehälter (4) eine Carbomerphase und in Ansatzbehälter (5) eine Parfümöl und Wirkstoffe enthaltende Phase.

Aus den Ansatzbehältern wird kontinuierlich dosiert. Die Ölphase aus Ansatzbehälter (1) wird zunächst mit der Wasserphase aus Ansatzbehälter (2) vereinigt, anschließend werden die Wasserphase aus Ansatzbehälter (2), NaOH-haltige Phase aus Ansatzbehälter (3) und die Carbomerphase aus Ansatzbehälter (4) zugegeben. Die Mischung durchläuft einen statischen Rohrmischer (10) Typ MS2G, Bran + Luebbe und wird anschließend in einem Schlaufenmischer (11) Typ Burdosa DMT 320 bei 1400 Umdrehungen pro Minute emulgiert. Dabei steigt die Temperatur auf 46,3°C. Die austretende Emulsion wird an Punkt (B) mit Kaltwasser schlagartig auf 31,1°C gekühlt und die Parfümöl und Wirkstoffe enthaltende Phase aus dem Ansatzbehälter (5) an Punkt (C) zugegeben. Nach dem Durchlaufen eines weiteren statischen Mischers (12), Typ MS2G, Bran + Luebbe, wird die Emulsion im Homogenisator (13) Typ Becomix DH 500, Firma Berents, homogenisiert, wobei die Temperatur auf 46,9°C ansteigt. Anschließend wird durch Wärmetauscher (14) auf 30°C abgekühlt und das Produkt abgefüllt. Es wird ein Durchsatz von 3,2 t/h erreicht.

### (3) Herstellung einer hautpflegenden Flüssigseife

| | | |
|---|---|---|
| Behälter 1 | 3,000 | Cocoamidopropylbetain |
| | 0,500 | Citronensäure |
| | 0,300 | PEG-40 hydriertes Rizinusöl |
| | | |
| Behälter 2 | 7,000 | Entsalztes Wasser |
| | 1,000 | Trinatrium EDTA 20%ige Lösung |
| | 0,500 | Acrylat Copolymer |
| | 0,450 | Natriumbenzoat |
| | 0,800 | PEG-200 hydriertes Glyceryl Palmölfettsäureester |
| | 4,000 | Dinatrium Kokosfettsäureglutamat |
| | | |
| Behälter 3 | 6,101 | Entsalztes Wasser |
| | 2,000 | Kochsalz |
| | | |
| Behälter 4 | 25,000 | Natriumlaurethsulfat |
| | | |
| Behälter 5 | 4,350 | Cocoamidopropylbetain |
| | 0,300 | Parfum |
| | 2,000 | Decylpolyglucose |
| | | |
| Kaltwasser | 6,000 | Entsalztes Wasser |
| | 2,000 | Kochsalz |

Zunächst werden folgende Phasen in Ansatzbehältern vorgelegt: in Ansatzbehälter (1) eine auf 40°C temperierte Ölphase, in Ansatzbehälter (2) eine auf 40°C temperierte Wasserphase, in Ansatzbehälter (3) eine elektrolythaltige Phase, in Ansatzbehälter (4) flüssiges Laurylethersulfat und in Ansatzbehälter (5) eine Parfümöl und Wirkstoffe enthaltende Phase. Aus den Ansatzbehältern wird kontinuierlich dosiert. Die Ölphase aus Ansatzbehälter (1) wird zunächst mit der Wasserphase aus Ansatzbehälter (2) vereinigt, anschließend werden die Wasserphase aus Ansatzbehälter (2), elektrolythaltige Phase aus Ansatzbehälter (3) und flüssiges Laurylethersulfat aus Ansatzbehälter (4) zugegeben. Die Mischung durchläuft einen statischen Rohrmischer (10) Typ MS2G, Bran + Luebbe und wird anschließend in einem Schlaufenmischer (11) Typ Burdosa DMT 320 bei 150 Umdrehungen pro Minute emulgiert. Dabei steigt die Temperatur auf 22,2°C. Die austretende Emulsion wird an Punkt (C) mit Kaltwasser schlagartig auf 18°C gekühlt und die Parfümöl und Wirkstoffe enthaltende Phase aus dem Ansatzbehälter (5) an Punkt (C) zugegeben. Nach dem Durchlaufen eines weiteren statischen Mischers (12), Typ MS2G, Bran + Luebbe, wird das WAS Produkt in einem Homogenisator (13) Typ Becomix DH 500, Firma Berents, homogenisiert, wobei die Temperatur auf 20,9°C ansteigt. Anschließend wird das Produkt abgefüllt. Es wird ein Durchsatz von 2,5 t/h erreicht.

### (4) Herstellung eines Sonnenschutzsprays (PIT-Emulsion)

| | | |
|---|---|---|
| Behälter 1 | 5,400 | Glycerylstearat + Ceteareth-20 +Ceteareth-12 + Cetearylalkohol |
| | 3,000 | Tridecyl Stearat (+) Tridecyl Trimellitat |
| | 3,300 | C12-15 Alkylbenzoat |
| | 0,500 | PVP/Hexadecen Copolymer |
| | 5,000 | Octylmethoxycinnamat |
| | 2,600 | Ceteareth-20 |
| | | |
| | 2,000 | Octyltriazon |
| | 1,000 | Diethylhexylbutamidotriazon |
| | 1,000 | Dicaprylylether |
| | | |
| Behälter 2 | 32,15 | Entsalztes Wasser |
| | 5,000 | Glycerin |
| | | |
| Behälter 3 | 7,800 | Entsalztes Wasser |
| | 0,150 | Natriumhydroxidlösung 45% |
| | 0, 500 | Phenylbenzimidazol Sulfonsäure |
| | | |
| Behälter 4 | 2,000 | Entsalztes Wasser |
| | 0,400 | DMDM Hydantoin |
| | | |
| Behälter 5 | 0,400 | Konservierungsstoffe |
| | 0,500 | Tocopherylacetat |
| | 0,300 | Parfum |
| | | |
| Kaltwasser | 27,000 | Entsalztes Wasser |

Zunächst werden folgende Phasen in Ansatzbehältern vorgelegt: in Ansatzbehälter (1) eine auf 60 bis 95°C temperierte Ölphase, in Ansatzbehälter (2) eine auf 60- bis 95°C temperierte Wasserphase, in Ansatzbehälter (3) eine elektrolythaltige Phase, in Ansatzbehälter (4) eine Konservierungsphase und in Ansatzbehälter (5) eine Parfümöl und Wirkstoffe enthaltende Phase. Aus den Ansatzbehältern wird kontinuierlich dosiert. Die Ölphase aus Ansatzbehälter (1) wird zunächst mit der Wasserphase aus Ansatzbehälter (2) vereinigt, anschließend werden die Wasserphase aus Ansatzbehälter (2), elektrolythaltige Phase aus Ansatzbehälter (3) und die Konservierungsphase aus Ansatzbehälter (4) zugegeben. Die Mischung durchläuft einen statischen Rohrmischer (10) Typ MS2G, Bran + Luebbe und wird anschließend in einem Schlaufenmischer (11) Typ Burdosa DMT 320 bei 1000 Umdrehungen pro Minute emulgiert. Die austretende Emulsion hat eine Temperatur von 93,1°C, wird an Punkt (B) mit Kaltwasser schlagartig auf 61,2°C gekühlt und die Parfümöl und Wirkstoffe enthaltende Phase aus dem Ansatzbehälter (5) an Punkt (C) zugegeben. Nach dem Durchlaufen eines weiteren statischen Mischers (12), Typ MS2G, Bran + Luebbe, wird die Emulsion in einem Homogenisator (13) Typ Becomix DH 500, Firma Berents, homogenisiert, wobei die Temperatur um 2 bis 10°C ansteigt. Anschließend wird durch Wärmetauscher (14) auf 28,6°C abgekühlt und das Produkt abgefüllt. Das Produkt hat eine Tröpfchengröße von 103,5 nm. Es wird ein Durchsatz von 3,5 t/h erreicht.

### (5) Herstellung einer Sonnenmilch

| | | |
|---|---|---|
| Behälter 1 | 5,500 | C12-15 Alkylbenzoat |
| | 4,160 | Glycerylstearat selbstemulgierend |
| | 2,500 | Capryl-/Caprin-Triglycerid |
| | 2,240 | Stearinsäure |
| | 0,750 | Cetearylether |
| | 3,000 | Octyltriazon |
| | 2,500 | Tocopherylacetat |
| | 5,500 | Octylmethoxycinnamat |
| | 1,000 | Titandioxid |
| | | |
| Behälter 2 | 14,806 | Entsalztes Wasser |
| | | |
| | 7, 500 | Glycerin |
| | 2,500 | Butylenglycol |
| | 0, 044 | Natriumhydroxidlösung 45% |
| | | |
| Behälter 3 | 19,300 | Entsalztes Wasser |
| | 2,000 | Dicaprylylether |
| | 0,500 | Xanthangummi |
| | | |
| Behälter 5 | 3,500 | Ethanol |
| | 0,300 | Konservierungsstoffe |
| | 2,000 | Capryl-/Caprin-Triglycerid |
| | 0,400 | Parfum |
| | | |
| Kaltwasser | 20,000 | Entsalztes Wasser |

Zunächst werden folgende Phasen in Ansatzbehältern vorgelegt: in Ansatzbehälter (1) eine auf 60 bis 95°C temperierte Ölphase, in Ansatzbehälter (2) eine auf 80°C temperierte Wasserphase, in Ansatzbehälter (3) eine Verdickerphase und in Ansatzbehälter (5) eine Parfümöl und Wirkstoffe enthaltende Phase. Aus den Ansatzbehältern wird kontinuierlich dosiert. Die Ölphase aus Ansatzbehälter (1) wird zunächst mit der Wasserphase aus Ansatzbehälter (2) vereinigt, anschließend werden die Wasserphase aus Ansatzbehälter (2), und die Verdickerphase aus Ansatzbehälter (3) zugegeben. Die Mischung durchläuft einen statischen Rohrmischer (10) Typ MS2G, Bran + Luebbe und wird anschließend in einem Schlaufenmischer (11) Typ Burdosa DMT 320 bei 1000 Umdrehungen pro Minute emulgiert. Die austretende Emulsion hat eine Temperatur von 46,2°C, wird an Punkt (B) mit Kaltwasser schlagartig auf 35-38°C gekühlt und die Parfümöl und Wirkstoffe enthaltende Phase aus dem Ansatzbehälter (5) an Punkt (C) zugegeben. Nach dem Durchlaufen eines weiteren statischen Mischers (12), Typ MS2G, Bran + Luebbe, wird die Emulsion in einem Homogenisator (13) Typ Becomix DH 500, Firma Berents, homogenisiert, wobei die Temperatur um 7 bis 11°C ansteigt. Anschließend wird durch Wärmetauscher (14) auf 30°C abgekühlt und das Produkt abgefüllt. Es wird ein Durchsatz von 2 t/h erreicht.

### (6) Herstellung einer wirkstoffhaltigen Creme

| | | |
|---|---|---|
| Behälter 2 | 45,178 | Vollentsalztes Wasser |
| | 7,500 | Glycerin |
| | 0,200 | Natriumhydroxidlösung 45% |
| | | |
| Behälter 1 | 3,000 | Glycerylstearatcitrat |
| | 4,000 | Caprylic/Capric Triglycerid |
| | 2,600 | Cetylalkohol |
| | | |
| Kaltwasser | 20,270 | Vollentsalztes Wasser |
| | | |
| Behälter 4 | 3,000 | Dicaprylylether |
| | 0,400 | Carbomer |
| | | |
| Behälter 3 | 0,002 | Active Ingredients |
| | 10,000 | Cyclomethicon |
| | | |
| Behälter 5 | 3,100 | Ethanol |
| | 0,500 | Preservatives |
| | 0,250 | Parfum |
| | | |

Zunächst werden folgende Phasen in Ansatzbehältern vorgelegt: in Ansatzbehälter (21) eine auf 60 bis 95°C temperierte Ölphase, in Ansatzbehälter (22) eine auf 60 bis 80°C temperierte Wasserphase, in Ansatzbehälter (23) eine elektrolythaltige Phase, in Ansatzbehälter (24) eine Carbomerphase und in Ansatzbehälter (25) eine Parfümöl und Wirkstoffe enthaltende Phase. Aus den Ansatzbehältern wird kontinuierlich dosiert. Die Ölphase aus Ansatzbehälter (21) wird zunächst mit der Wasserphase aus Ansatzbehälter (22) vereinigt und durchläuft einen statischen Rohrmischer (28) Typ MS2G, Bran + Luebbe anschließend werden die wirkstoffhaltige Phase aus Ansatzbehälter (23) und die Carbomerphase aus Ansatzbehälter (24) zugegeben. Die Mischung durchläuft den Homogenisator Typ Becomix DH 500, Firma Berents (29) mit 800 Umdrehungen pro Minute und wird anschließend in einem Schlaufenmischer (30) Typ Burdosa DMT 320 bei 500 Umdrehungen pro Minute emulgiert. Die austretende Emulsion hat eine Temperatur von 53,1°C, wird an Punkt (Q) mit Kaltwasser schlagartig auf 35-38°C gekühlt. Nach dem Durchlaufen eines weiteren statischen Mischers (31), Typ MS2G, Bran + Luebbe und einer Kühlung auf 31 °C im Wärmetauscher (32) wird die Parfümöl und Wirkstoffe enthaltende Phase aus dem Ansatzbehälter (25) an Punkt (S) zugegeben,. Danach wird die Emulsion in einem Homogenisator (33) Typ Becomix DH 500, Firma Berents, mit 2000 Umdrehungen pro Minute homogenisiert, wobei die Temperatur um 2 bis 10°C ansteigt. Anschließend wird durch Wärmetauscher (34) auf 28°C abgekühlt und das Produkt abgefüllt. Es wird ein Durchsatz von 6 t/h erreicht.

### (7) Herstellung einer Softcreme

| | | |
|---|---|---|
| Behälter 1 | 3,500 | Paraffinöl |
| | 1,500 | Stearinsäure |
| | 0,500 | Myristylmyristat |
| | 1,100 | Hydryierte Kokosfettglyceride |
| | 0,350 | Konservierungsstoffe |
| | | |
| Behälter 2 | 29,130 | Entsalztes Wasser |
| | 3,500 | Glycerin |
| | | |
| Behälter 3 | 6,000 | Entsalztes Wasser |
| | 0,600 | Natriumhydroxidlösung 45% |
| | | |
| Behälter 4 | 0,750 | Dimethicon |
| | 0,300 | Carbomer |
| | 18,750 | Entsalztes Wasser |
| | 2,800 | Ethanol, denaturiert |
| | | |
| Behälter 5 | 0,500 | Actives |
| | 0,350 | Silicon Oil |
| | 0,200 | Ethanol |
| | 0,170 | Parfum |
| | | |
| Kaltwassser | 30,000 | Entsalztes Wasser |

Zunächst werden folgende Phasen in Ansatzbehältern vorgelegt: in Ansatzbehälter (21) eine auf 60 bis 95°C temperierte Ölphase, in Ansatzbehälter (22) eine auf 80°C temperierte Wasserphase, in Ansatzbehälter (23) eine NaOH-haltige Phase, in Ansatzbehälter (24) eine Carbomerphase und in Ansatzbehälter (25) eine Parfümöl und Wirkstoffe enthaltende Phase. Aus den Ansatzbehältern wird kontinuierlich dosiert.

Die Ölphase aus Ansatzbehälter (21) wird zunächst mit der Wasserphase ansatzbehälter (22) vereinigt und durchläuft einen statischen Rohrmischer (28) Typ MS2G, Bran + Luebbe, anschließend werden die NaOH-haltige Phase aus Ansatzbehälter (23) und die Carbomerphase aus Ansatzbehälter (24) zugegeben. Kurz vor dem Homogenisator (29) Typ Becomix DH 500, Firma Berents wird an Punkt (P) mit Kaltwasser schlagartig auf 38-41 °C gekühlt. Die Mischung durchläuft den Homogenisator Typ Becomix DH 500, Firma Berents bei 1000 Umdrehungen (29) und wird anschließend in einem Schlaufenmischer (30) Typ Burdosa DMT 320 bei 1400 Umdrehungen pro Minute emulgiert. Dabei stellt sich eine Temperatur auf 42,3°C ein. Nach dem Durchlaufen eines weiteren statischen Mischers (31), Typ MS2G, Bran + Luebbe und einer Kühlung in Wärmetauscher (32) wird die Parfümöl und Wirkstoffe enthaltende Phase aus dem Ansatzbehälter (25) an Punkt (R) zugegeben, Danach wird die Emulsion in einem Homogenisator (33) Typ Becomix DH 500, Firma Berents, mit 1000 Umdrehungen pro Minute homogenisiert, wobei die Temperatur um 2 bis 10°C ansteigt. Anschließend wird durch Wärmetauscher (34) auf 30°C abgekühlt und das Produkt abgefüllt. Es wird ein Durchsatz von 6 t/h erreicht.

### (8) Herstellung einer hautpflegenden Flüssigseife

| | | |
|---|---|---|
| Behälter 1 | 3,500 | Cocoamidopropylbetain |
| | 0,300 | PEG-40 hydriertes Rizinusöl |
| | | |
| Behälter 2 | 8,000 | Entsalztes Wasser |
| | 0,500 | Acrylat Copolymer |
| | 0,450 | Preservatives |
| | 4,800 | Dinatrium Kokosfettsäureglutamat |
| | | |
| Behälter 3 | 6,101 | Entsalztes Wasser |
| | 2,000 | Kochsalz |
| | | |
| Behälter 4 | 25,000 | Natriumlaurethsulfat |
| | | |
| Behälter 5 | 6,350 | Cocoamidopropylbetain |
| | 0,300 | Parfum |
| | | |
| Kaltwasser | 40,699 | Entsalztes Wasser |

Zunächst werden folgende Phasen in Ansatzbehältern vorgelegt: in Ansatzbehälter (21) eine auf 40°C temperierte Ölphase, in Ansatzbehälter (22) eine auf 40°C temperierte Wasserphase, in Ansatzbehälter (23) eine elektrolythaltige Phase, in Ansatzbehälter (24) flüssiges Larylethersulfat und in Ansatzbehälter (25) eine Parfümöl und Wirkstoffe enthaltende Phase. Aus den Ansatzbehältern wird kontinuierlich dosiert. Die Ölphase aus Ansatzbehälter (21) wird zunächst mit der Wasserphase aus Ansatzbehälter (22) vereinigt, und durchläuft einen statischen Rohrmischer (28) Typ MS2G, Bran + Luebbe anschließend werden die elektrolythaltige Phase aus Ansatzbehälter (23) und flüssiges Laurylethersulfat aus Ansatzbehälter (24) zugegeben. Die Mischung durchläuft den Homogenisator Typ Becomix DH 500, Firma Berents (29) mit lediglich 500 Umdrehungen pro Minute und wird anschließend in einem Schlaufenmischer (30) Typ Burdosa DMT 320 bei 1000 Umdrehungen pro Minute intensiv gemischt. Dabei steigt die Temperatur auf 22,2°C. Die austretende Emulsion wird an Punkt (Q) mit Kaltwasser schlagartig auf 18°C gekühlt und die Parfümöl und Wirkstoffe enthaltende Phase aus dem Ansatzbehälter (25) an Punkt (R) zugegeben. Nach dem Durchlaufen eines weiteren statischen Mischers (31), Typ MS2G, Bran + Luebbe) wird die Parfümöl und Wirkstoffe enthaltende Phase aus dem Ansatzbehälter (25) an Punkt (S) zugegeben. Die Wärmetauscher (32, 34) haben hierbei keine Funktion. Abschließend wird das WAS Produkt in einem Homogenisator (33) Typ Becomix DH 500, Firma Berents mit 1000 Umdrehungen pro Minute, homogenisiert, wobei die Temperatur auf 20,9°C ansteigt. Anschließend wird das Produkt abgefüllt. Es wird ein Durchsatz von 8 t/h erreicht.

### (9) Herstellung eines Sonnenschutzsprays (PIT-Emulsion)

| | | |
|---|---|---|
| Behälter 1 | 5,400 | Glycerylstearat + Ceteareth-20 +Ceteareth-12 + Cetearylalkohol |
| | 3,000 | Tridecyl Stearat (+) Tridecyl Trimellitat |
| | 3,300 | C12-15 Alkylbenzoat |
| | 3,100 | Paraffin Oil |
| | 7,000 | Octylmethoxycinnamat |
| | 1,000 | Diethylhexylbutamidotriazon |
| | | |
| Behälter 2 | 33,15 | Entsalztes Wasser |
| | 5,000 | Glycerin |
| | | |
| Behälter 3 | 7,800 | Entsalztes Wasser |
| | 0,150 | Natriumhydroxidlösung 45% |
| | 0,500 | Phenylbenzimidazol Sulfonsäure |
| | | |
| Behälter 4 | 2,000 | Entsalztes Wasser |
| | 0,400 | Preservatives |
| | | |
| Behälter 5 | 0,400 | Preservatives |
| | 0,500 | Active Ingredients |
| | 0,300 | Parfum |
| | | |
| Kaltwasser | 27,000 | Entsalztes Wasser |

Zunächst werden folgende Phasen in Ansatzbehältern vorgelegt: in Ansatzbehälter (21) eine auf 60 bis 95°C temperierte Ölphase, in Ansatzbehälter (22) eine auf 60- bis 95°C temperierte Wasserphase, in Ansatzbehälter (23) eine elektrolythaltige Phase, in Ansatzbehälter (4) eine Konservierungsphase und in Ansatzbehälter (25) eine Parfümöl und Wirkstoffe enthaltende Phase. Aus den Ansatzbehältern wird kontinuierlich dosiert. Die Ölphase aus Ansatzbehälter (21) wird zunächst mit der Wasserphase aus Ansatzbehälter (22) vereinigt, und durchläuft einen statischen Rohrmischer (28) Typ MS2G, Bran + Luebbe anschließend werden die elektrolythaltige Phase aus Ansatzbehälter (23) und die Konservierungsphase aus Ansatzbehälter (24) zugegeben Die Mischung durchläuft zwar den Homogenisator Typ Becomix DH 500, Firma Berents (29). Dieser ist jedoch für diesen Emulsionstyp abgeschaltet. Danach wird die Emulsion in einem Schlaufenmischer (31) Typ Burdosa DMT 320 bei 1000 Umdrehungen pro Minute emulgiert Die austretende Emulsion hat eine Temperatur von 93,1°C, und wird an Punkt (Q) mit Kaltwasser schlagartig auf 61,2°C gekühlt. Die Mischung durchläuft einen statischen Rohrmischer (30) Typ MS2G, Bran + Luebbe und anschließend. wird Parfümöl und Wirkstoffe enthaltende Phase aus dem Ansatzbehälter (25) an Punkt (R) zugegeben. Nach dem Durchlaufen eines Wärmetauschers (32), wobei sich die Temperatur auf 38 °C einstellt, wird die Emulsion in einem Homogenisator (33) Typ Becomix DH 500, Firma Berents, bei 500 Umdrehungen pro Minute homogenisiert, wobei die Temperatur nicht ansteigt. Anschließend wird durch Wärmetauscher (34) auf 28,6°C abgekühlt und das Produkt abgefüllt. Das Produkt hat eine Tröpfchengröße von 103,5 nm. Es wird ein Durchsatz von 7 t/h erreicht.

### (10) Herstellung einer Sonnenmilch

| | | |
|---|---|---|
| Behälter 1 | 8,500 | Paraffin oil |
| | 4,160 | Glycerylstearat selbstemulgierend |
| | 2,500 | Capryl-/Caprin-Triglycerid |
| | 2,240 | Stearinsäure |
| | 0,750 | Cetearylalkohol |
| | 2,500 | Active Ingredients |
| | 5,500 | Octylmethoxycinnamat |
| | 1,000 | Titandioxid |
| | | |
| Behälter 2 | 22,306 | Entsalztes Wasser |
| | | |
| | 2,500 | Butylenglycol |
| | 0,044 | Natriumhydroxidlösung 45% |
| | | |
| Behälter 3 | 19,300 | Entsalztes Wasser |
| | 2,000 | Dicaprylylether |
| | 0,500 | Xanthangummi |
| | | |
| Behälter 5 | 3,500 | Ethanol |
| | 0,300 | Konservierungsstoffe |
| | 2,000 | Capryl-/Caprin-Triglycerid |
| | 0,400 | Parfum |
| | | |
| Kaltwasser | 20,000 | Entsalztes Wasser |

Zunächst werden folgende Phasen in Ansatzbehältern vorgelegt: in Ansatzbehälter (21) eine auf 60 bis 95°C temperierte Ölphase, in Ansatzbehälter (22) eine auf 60 bis 80°C temperierte Wasserphase, in Ansatzbehälter (23) eine Verdickerphase und in Ansatzbehälter (25) eine Parfümöl und Wirkstoffe enthaltende Phase. Aus den Ansatzbehältern wird kontinuierlich dosiert. Die Ölphase aus Ansatzbehälter (21) wird zunächst mit der Wasserphase aus Ansatzbehälter (22) vereinigt, und durchläuft einen statischen Rohrmischer (28) Typ MS2G, Bran + Luebbe, anschließend wird die Verdickerphase aus Ansatzbehälter (23) zugegeben. Die Mischung durchläuft den Homogenisator Typ Becomix DH 500, Firma Berents mit 1200 Umdrehungen pro Minute (29) und wird anschließend in einem Schlaufenmischer (30) Typ Burdosa DMT 320 bei 1200 Umdrehungen pro Minute emulgiert. Die austretende Emulsion hat eine Temperatur von 46,2°C und wird an Punkt (Q) mit Kaltwasser schlagartig auf 35-38°C gekühlt. Nach dem Durchlaufen eines weiteren statischen Mischers (31), Typ MS2G, Bran + Luebbe und einer Kühlung im Wärmetauscher (32) wird die Parfümöl und Wirkstoffe enthaltende Phase aus dem Ansatzbehälter (25) an Punkt (S) zugegeben. Danach wird die Emulsion in einem Homogenisator (13) Typ Becomix DH 500, Firma Berents, bei 2000 Umdrehungen pro Minute homogenisiert, wobei die Temperatur um 2 bis 10°C ansteigt. Anschließend wird durch Wärmetauscher (14) auf 30°C abgekühlt und das Produkt abgefüllt. Es wird ein Durchsatz von 6 t/h erreicht.

## Patentansprüche

1. Herstellverfahren für kosmetische und/oder dermatologische Zubereitungen, die temperaturempfindliche Inhaltstoffe enthalten, **gekennzeichnet dadurch, dass**
(1) es kontinuierlich durchgeführt wird sowie
(2) durch eine Abfolge folgender Verfahrensschritte
(a) Emulgieren in Mischapparaten (30), in Kombination mit statischen Mischern (28, 11) bzw. Homogenisatoren (29, 33),
(b) Einstellen einer Temperatur der Mischung von 55 - 35°C durch Zugabe (P,Q) von wässriger Phase von 15 - 50°C mit einer gegenüber der Mischung niedrigeren Temperatur,
(c) Zugabe (R,S) von Parfümöl und/oder temperaturempfindlichen Wirkstoffen bei unterschiedlichen Temperaturen,
(d) Homogenisieren in Apparaten (29, 33) im Temperaturbereich von entweder 50 bis 80°C. besonders bevorzugt bei 60 bis 70°C oder 28 bis 50 °C, besonders bevorzugt 28 bis 45°C ganz besonders bevorzugt 30 bis 40°C,
(e) Schrittweises (stufenweises) Kühlen währendes des Prozesses (32,34).

2. Verfahren nach Anspruch 1 **dadurch gekennzeichnet, dass** die eintretenden Vorprodukte zuvor auf Temperaturen von 40 bis 100°C, besonders bevorzugt bei 50 bis 90°C temperiert wurden.

3. Herstellverfahren für kosmetische und/oder dermatologische Zubereitungen, die temperaturempfindliche Inhaltstoffe enthalten, **gekennzeichnet dadurch, dass**
(1) es kontinuierlich durchgeführt wird sowie
(2) durch eine Abfolge folgender Verfahrensschritte
(a) Emulgieren in Mischapparaten (11),
(b) Einstellen einer Temperatur der Mischung von weniger 40°C durch Zugabe (B) von wässriger Phase mit einer gegenüber der Mischung niedrigeren Temperatur,
(c) Zugabe (C) von Parfümöl und/oder temperaturempfindlichen Wirkstoffen,
(d1) Homogenisieren in Apparaten (13) im Temperaturbereich von 20 bis 50°C, besonders bevorzugt bei 28 bis 40°C.

4. Verfahren nach mindestens einem der vorangehenden Ansprüche **dadurch gekennzeichnet, dass** als weiterer nachgeschalteter Verfahrensschritt (14) oder (32, 34) das Verfahrensprodukt auf höchstens 28 bis 30°C gekühlt wird.

5. Verfahren nach mindestens einem der vorangehenden Ansprüche **dadurch gekennzeichnet, daß** die Vorprodukte bei Temperaturen von 40 bis 100°C, besonders bevorzugt bei 50 bis 90°C vermischt werden, bevor sie in den ersten Misch- oder Homogenisierapparat eintreten.

6. Herstellverfahren für kosmetische und/oder dermatologische Zubereitungen nach mindestens einem der vorangehenden Ansprüche, **dadurch gekennzeichnet, dass** beim Durchlaufen des Homogenisierapparates (13) die Temperatur der austretenden Mischung 2 bis 60°C oder beim Durchlaufen des Homogenisierapparates (29, 33) die Temperatur der austretenden Mischung 2 bis 10°C ansteigt, bezogen auf die Temperatur der eintretenden Mischung.

7. Herstellverfahren für kosmetische und/oder dermatologische Zubereitungen nach mindestens einem der vorangehenden Ansprüche, **dadurch gekennzeichnet, dass** der Emulgiervorgang in zwei unterschiedlichen Mischapparaten (10) und (11) oder (29) und (33) durchgeführt wird,

8. Herstellverfahren für kosmetische und/oder dermatologische Zubereitungen nach mindestens einem der vorangehenden Ansprüche, **dadurch gekennzeichnet, dass** der Emulgiervorgang in einem statischen Mischer (10) und einem Schlaufenmischer (11) durchgeführt wird.

9. Herstellverfahren für kosmetische und/oder dermatologische Zubereitungen nach mindestens einem der vorangehenden Ansprüche, **dadurch gekennzeichnet, dass** der Homogenisiervorgang in einem Schlaufenmischer (30) und einem Homogenisator (33) durchgeführt wird.

10. Herstellverfahren für kosmetische und/oder dermatologische Zubereitungen nach mindestens einem der vorangehenden Ansprüche, **dadurch gekennzeichnet, dass** der Homogenisiervorgang in zwei unterschiedlichen Apparaten (12) und (13) durchgeführt wird.

11. Herstellverfahren für kosmetische und/oder dermatologische Zubereitungen nach mindestens einem der vorangehenden Ansprüche, **dadurch gekennzeichnet, dass** der Homogenisiervorgang in einem statischen Mischer (12) und einem Homogenisator (13) durchgeführt wird.

12. Herstellverfahren für kosmetische und/oder dermatologische Zubereitungen nach mindestens einem der vorangehenden Ansprüche, **dadurch gekennzeichnet, dass** der Emulgiervorgang in einem Schlaufenmischer (30) in Kombination mit einem oder mehreren Homogenisatoren (29, 33), sowie statischen Mischern (28, 31) durchgeführt wird.

13. Emulsionen, PIT-Emulsionen und waschaktive Substanzen enthaltende Produkte erhältlich durch ein Verfahren nach mindestens einem der vorangehenden Ansprüche.

14. Emulsionen, PIT-Emulsionen und waschaktive Substanzen enthaltende Produkte nach Anspruch 9 sowie Verfahren nach einem der Ansprüche 1 bis 9 **dadurch gekennzeichnet, dass** die Temperatur empfindlichen inhaltsstoffe Duftstoffe, Vitamine, Co-Enzyme, Peptide, Enzyme, Nukleinsäuren, Pflanzenextrakte und/oder Konservierungsstoffe sind.

## Claims

1. Preparation process for cosmetic and/or dermatological preparations which comprise temperature-sensitive ingredients, **characterized in that**
(1) it is carried out continuously and
(2) by a sequence of the following process steps
(a) emulsification in mixing apparatuses (30), in combination with static mixers (28, 11) and/or homogenizers (29, 33),
(b) establishing a mixture temperature of 55-35°C by adding (P,Q) aqueous phase of 15-50°C with a lower temperature compared with the mixture,
(c) addition (R,S) of perfume oil and/or temperature-sensitive active ingredients at different temperatures,
(d) homogenization in apparatuses (29, 33) in the temperature range from either 50 to 80°C, particularly preferably 60 to 70°C or 28 to 50°C, particularly preferably 28 to 45°C, very particularly preferably 30 to 40°C,
(e) stepwise (stagewise) cooling during the process (32, 34) .

2. Process according to Claim 1, **characterized in that** the entering preproducts have been heated beforehand to temperatures of from 40 to 100°C, particularly preferably 50 to 90°C.

3. Preparation process for cosmetic and/or dermatological preparations which comprise temperature-sensitive ingredients, **characterized in that**
(1) it is carried out continuously and
(2) by a sequence of the following process steps
(a) emulsification in mixing apparatuses (11),
(b) establishing a mixture temperature of less than 40°C by adding (B) aqueous phase with a lower temperature compared with the mixture,
(c) addition (C) of perfume oil and/or temperature-sensitive active ingredients,
(d1) homogenization in apparatuses (13) in the temperature range from 20 to 50°C, particularly preferably 28 to 40°C.

4. Process according to at least one of the preceding claims, **characterized in that**, as a further downstream process step (14) or (32, 34), the process product is cooled to at most 28 to 30°C.

5. Process according to at least one of the preceding claims, **characterized in that** the preproducts are mixed at temperatures of from 40 to 100°C, particularly preferably at 50 to 90°C before they enter the first mixing or homogenization apparatus.

6. Preparation process for cosmetic and/or dermatological preparations according to at least one of the preceding claims, **characterized in that**, upon passing through the homogenization apparatus (13), the temperature of the exiting mixture increases 2 to 60°C or upon passing through the homogenization apparatus (29, 33) the temperature of the exiting mixture increases 2 to 10°C, based on the temperature of the entering mixture.

7. Preparation process for cosmetic and/or dermatological preparations according to at least one of the preceding claims, **characterized in that** the emulsification operation is carried out in two different mixing apparatuses (10) and (11) or (29) and (33).

8. Preparation process for cosmetic and/or dermatological preparations according to at least one of the preceding claims, **characterized in that** the emulsification operation is carried out in a static mixer (10) and a loop mixer (11).

9. Preparation process for cosmetic and/or dermatological preparations according to at least one of the preceding claims, **characterized in that** the homogenization operation is carried out in a loop mixer (30) and a homogenizer (33).

10. Preparation process for cosmetic and/or dermatological preparations according to at least one of the preceding claims, **characterized in that** the homogenization operation is carried out in two different apparatuses (12) and (13).

11. Preparation process for cosmetic and/or dermatological preparations according to at least one of the preceding claims, **characterized in that** the homogenization operation is carried out in a static mixer (12) and a homogenizer (13).

12. Preparation process for cosmetic and/or dermatological preparations according to at least one of the preceding claims, **characterized in that** the emulsification operation is carried out in a loop mixer (30) in combination with one or more homogenizers (29, 33), and static mixers (28, 31).

13. Emulsions, PIT emulsions and products comprising washing-active substances, obtainable by a process according to at least one of the preceding claims.

14. Emulsions, PIT emulsions and products comprising washing-active substances according to Claim 9, and process according to one of Claims 1 to 9, **characterized in that** the temperature-sensitive ingredients are fragrances, vitamins, coenzymes, peptides, enzymes, nucleic acids, plant extracts and/or preservatives.

## Revendications

1. Procédé de fabrication de préparations cosmétiques et/ou dermatologiques qui contiennent des composants sensibles à la température, **caractérisé en ce que**
(1) il est effectué en continu, ainsi que
(2) par une succession des étapes suivantes de processus
(a) émulsification dans des appareils de mélange (30) en combinaison avec des mélangeurs statiques (28, 11) ou des homogénéisateurs (29, 33),
(b) ajustement d'une température du mélange de 55-35 °C par addition (P, Q) de phase aqueuse à 15-50 °C, ayant une température plus basse vis-à-vis du mélange,
(c) addition (R, S) d'huile essentielle et/ou de substances actives sensibles à la température, à différentes températures,
(d) homogénéisation dans des appareils (29, 33) dans la plage de température allant soit de 50 à 80 °C, de façon particulièrement préférée de 60 à 70 °C, soit de 28 à 50 °C, de façon particulièrement préférée de 28 à 45 °C, de façon tout particulièrement préférée de 30 à 40 °C,
(e) refroidissement graduel (par étapes) pendant le processus (32, 34).

2. Procédé selon la revendication 1, **caractérisé en ce que** les précurseurs introduits ont été portés au préalable à des températures de 40 à 100 °C, de façon particulièrement préférée de 50-90 °C.

3. Procédé de fabrication de préparations cosmétiques et/ou dermatologiques qui contiennent des composants sensibles à la température, **caractérisé en ce que**
(1) il est effectué en continu, ainsi que
(2) par une succession des étapes suivantes de processus
(a) émulsification dans des appareils de mélange (11),
(b) ajustement d'une température du mélange inférieure à 40 °C par addition (B) de phase aqueuse à une température plus basse vis-à-vis du mélange,
(c) addition (C) d'huile essentielle et/ou de substances actives sensibles à la température,
(d1) homogénéisation dans des appareils (13) dans la plage de température allant de 20 à 50 °C, de façon particulièrement préférée de 28 à 40 °C.

4. Procédé selon au moins l'une des revendications précédentes, **caractérisé en ce qu'**en tant qu'autre étape (14) ou (32, 34) du processus, effectuée ensuite, le produit du processus est refroidi jusqu'à 28 à 30°C au maximum.

5. Procédé selon au moins l'une des revendications précédentes, **caractérisé en ce que** les précurseurs sont mélangés à des températures de 40 à 100 °C, de façon particulièrement préférée de 50 à 90 °C, avant d'être introduits dans le premier appareil de mélange ou d'homogénéisation.

6. Procédé de fabrication de préparations cosmétiques et/ou dermatologiques selon au moins l'une des revendications précédentes, **caractérisé en ce que** lors de la traversée de l'appareil de mélange (13), la température du mélange sortant s'élève de 2 à 60 °C ou lors de la traversée de l'appareil d'homogénéisation (29, 33), la température du mélange sortant s'élève de 2 à 10 °C, par rapport à la température du mélange entrant.

7. Procédé de fabrication de préparations cosmétiques et/ou dermatologiques selon au moins l'une des revendications précédentes, **caractérisé en ce que** le processus d'émulsification est effectué dans deux appareils de mélange différents (10) et (11) ou (29) et (33).

8. Procédé de fabrication de préparations cosmétiques et/ou dermatologiques selon au moins l'une des revendications précédentes, **caractérisé en ce que** le processus d'émulsification est effectué dans un mélangeur statique (10) et un mélangeur à circulation (11).

9. Procédé de fabrication de préparations cosmétiques et/ou dermatologiques selon au moins l'une des revendications précédentes, **caractérisé en ce que** le processus d'homogénéisation est effectué dans un mélangeur à circulation (30) et un homogénéisateur (33) .

10. Procédé de fabrication de préparations cosmétiques et/ou dermatologiques selon au moins l'une des revendications précédentes, **caractérisé en ce que** le processus d'homogénéisation est effectué dans deux appareils différents (12) et (13).

11. Procédé de fabrication de préparations cosmétiques et/ou dermatologiques selon au moins l'une des revendications précédentes, **caractérisé en ce que** le processus d'homogénéisation est effectué dans un mélangeur statique (12) et un homogénéisateur (13).

12. Procédé de fabrication de préparations cosmétiques et/ou dermatologiques selon au moins l'une des revendications précédentes, **caractérisé en ce que** le processus d'émulsification est effectué dans un mélangeur à circulation (30) en combinaison avec un ou plusieurs homogénéisateurs (29, 33) et mélangeurs statiques (28, 31).

13. Produits contenant des émulsions, des émulsions PIT et des substances lavantes, pouvant être obtenus par un procédé selon au moins l'une des revendications précédentes.

14. Produits contenant des émulsions, des émulsions PIT et des substances lavantes, selon la revendication 9 ainsi que procédé selon l'une quelconque des revendications 1 à 9, **caractérisés en ce que** les composants sensibles à la température sont des parfums, des vitamines, des co-enzymes, des peptides, des enzymes, des acides nucléiques, des extraits de plantes et/ou des conservateurs.
